# FASCICULE DE BREVET EUROPEEN

(11) **EP 0 682 011 B1**
(45) Date de publication et mention de la délivrance du brevet: **23.04.1997**
(21) Numéro de dépôt: 95400576.5
(22) Date de dépôt: 15.03.1995
(51) Int. Cl.: C07C 271/22, A61K 7/48

(54) **Nouveaux dérivés de la sérine, leur préparation et leur utilisation dans des compositions cosmétiques ou dermatologiques**
Neue Serinderivate, ihre Herstellung und ihre Verwendung in kosmetischen und dermatologischen Zusammensetzungen
New derivatives of serine, their preparation and their use in cosmetic and dermatologic compositions

(30) Priorité: 09.05.1994 FR 9405689
(43) Date de publication de la demande: 15.11.1995
(73) Titulaire: L'OREAL, 75008 Paris (FR)
(72) Inventeur: Philippe, Michel, F-91320 Wissous (FR)
(74) Mandataire: Dodin, Catherine

(56) Documents cités:
- EP-A- 0 408 448
- EP-A- 0 577 506
- WO-A-86/03746

## Description

L'invention a pour objet de nouveaux dérivés de la sérine et leur préparation. Elle a également pour objet des compositions cosmétiques ou dermatologiques contenant ces nouveaux dérivés.

La sérine est un acide aminé important pour les cheveux et pour la peau, car c'est un précurseur des céramides, composés du stratum corneum de l'épiderme et des cheveux, jugés essentiels par leur propriétés de barrière empêchant par exemple les problèmes de perméabilité à l'eau.

On connaît d'après la demande de brevet EP-A-0408448 des dérivés de la sérine qui sont des lipoaminoacides à fonction uréthane. Il s'agit de composés ioniques à une seule chaîne, entrant dans des compositions cosmétiques ou dermatologiques pour le traitement de la peau, donnant d'excellents résultats sur les peaux sèches.

La demanderesse a trouvé de nouveaux dérivés de la sérine qui ont au moins les mêmes propriétés pour la peau que ceux décrits dans la demande de brevet EP-A-0408448, mais présentent en plus, un grand intérêt dans le traitement du cheveu comme agent démêlant ou de lissage.

L'invention a donc pour objet des dérivés de la sérine caractérisés par le fait qu'ils répondent à la formule générale : 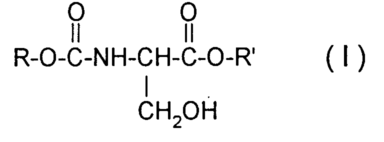 dans laquelle R et R', identiques ou différents, représentent un radical hydrocarboné linéaire ou ramifié ayant de 8 à 30 atomes de carbone,
et leurs isomères optiques D ou L ou leurs mélanges.

Par radical hydrocarboné, on entend tout radical hydrocarboné saturé ou insaturé ayant de 8 à 30 atomes de carbone.

Selon un mode de réalisation préférentiel de l'invention, R et R', identiques ou différents, sont des radicaux saturés ou qui comportent une double liaison.

Selon un autre mode de réalisation de l'invention, R et R', identiques ou différents, contiennent de préférence entre 8 et 18 atomes de carbone.

On peut citer comme composés de formule (I) :
- le 3-hydroxy-2-(N-hexadécyloxycarbonyl)-aminopropionate de dodécyle,
- le 3-hydroxy-2-(N-hexadécyloxycarbonyl)-aminopropionate d'octyle,
- le 3-hydroxy-2-(N-hexadécyloxycarbonyl)-aminopropionate d'hexadécyle,
- le 3-hydroxy-2-(N-2-éthyl-hexyloxycarbonyl)-aminopropionate de dodécyle,
- le 3-hydroxy-2-(N-2-éthyl-hexyloxycarbonyl)-aminopropionate d'octyle,
- le 3-hydroxy-2-(N-2-éthyl-hexyloxycarbonyl)-aminopropionate d'hexadécyle,
- le 3-hydroxy-2-(N-dodécyloxycarbonyl)-aminopropionate de dodécyle,
- le 3-hydroxy-2-(N-dodécyloxycarbonyl)-aminopropionate d'octyle,
- le 3-hydroxy-2-(N-dodécyloxycarbonyl)-aminopropionate d'hexadécyle,
- le 3-hydroxy-2-(N-oleyloxycarbonyl)-aminopropionate de dodécyle,
- le 3-hydroxy-2-(N-oleyloxycarbonyl)-aminopropionate d'octyle,
- le 3-hydroxy-2-(N-oleyloxycarbonyl)-aminopropionate d'hexadécyle.

Parmi ces composés on préfère tout particulièrement :
- le 3-hydroxy-2-(N-hexadécyloxycarbonyl)-aminopropionate de dodécyle,
- le 3-hydroxy-2-(N-hexadécyloxycarbonyl)-aminopropionate d'octyle,
- le 3-hydroxy-2-(N-hexadécyloxycarbonyl)-aminopropionate d'hexadécyle.

Un deuxième objet de l'invention est un procédé de préparation des composés de formule (I) tels que définis ci-dessus.

Ce procédé est caractérisé par le fait que l'on fait réagir, en présence d'un agent de transfert de phase, un sel de N-liposérine répondant à la formule : 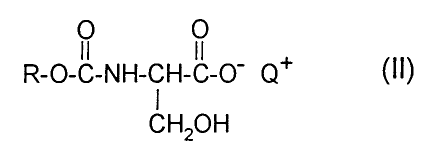 et un dérivé hydrocarboné activé répondant à la formule :

R'X (III)

dans lesquelles
R et R', identiques ou différents, représentent un radical hydrocarboné linéaire ou ramifié, ayant de 8 à 30 atomes de carbone ;
Q⁺ est un cation organique ou minéral ;
et X un groupement activant.

Selon un mode de réalisation préférentiel de l'invention R et R', identiques ou différents sont des radicaux saturés ou qui comportent une double liaison.

Selon un autre mode de réalisation de l'invention R et R', identiques ou différents, contiennent de préférence entre 8 et 18 atomes de carbone.

On peut citer comme exemple de cation Q⁺ les cations alcalins comme le potassium.

Le groupement activant X peut être un atome d'halogène ou un radical mésyloxy, brosyloxy ou tosyloxy.

On peut utiliser comme agent de transfert de phase tout composé habituellement utilisé dans ce type de réaction. Préférentiellement, on utilise un agent de transfert de phase solide/liquide. On mettra en oeuvre de préférence un dérivé d'ammonium quaternaire comme par exemple le chlorure de trioctylméthylammonium.

Les sels de liposérine utilisés dans ce procédé sont, par exemple, les sels des composés cités dans la demande de brevet EP-A-0408448, comme les sels de la N-hexadécyloxycarbonyl-sérine, la N-(éthyl-2-hexyloxycarbonyl)-sérine ou la N-dodécyloxycarbonyl-sérine. Ce peut être également les sels de la N-oleyloxycarbonyl-sérine.

Les sels de liposérines utilisés peuvent se présenter sous la forme de leurs isomères D ou L, ou d'un mélange de ces formes.

Selon un premier mode de réalisation du procédé de l'invention, (A), la réaction de transfert de phase a lieu à une température supérieure à 20°C, de préférence comprise entre 60°C et 180°C et encore plus préférentiellement entre 80°C et 120°C. La durée de la réaction est alors supérieure à 2 heures, préférentiellement comprise entre 2 et 15 heures et encore plus préférentiellement entre 4 et 10 heures.

Selon un deuxième mode de réalisation du procédé selon l'invention (B), la réaction de transfert de phase peut avoir lieu en soumettant le milieu réactionnel, dans l'enceinte d'un four à micro-ondes, à une puissance nominale supérieure à 100 watts pendant une durée réactionnelle de 1 à 15 minutes et de préférence entre 2 et 7 minutes.

Quel que soit le mode de préparation utilisé, (A ou B), le rapport molaire du composé de formule III au composé de formule II est compris entre 1 et 5. De manière préférentielle, ce rapport est compris entre 1,2 et 2.

L'agent de transfert de phase est, dans le milieu réactionnel, quel que soit le mode opératoire utilisé, en une proportion comprise entre 0,1% et 10% et de préférence entre 0,5% et 5%, en poids par rapport au poids de composé de formule I.

Une fois la réaction terminée, les sels peuvent être éliminés par lavage à l'eau. L'ester ainsi obtenu est ensuite purifié et caractérisé selon les techniques connues de l'homme de l'art.

Un troisième objet de l'invention concerne des compositions cosmétiques ou dermatologiques, contenant au moins un des dérivés de la sérine répondant à la formule (I).

Dans les compositions selon l'invention, les dérivés de formule (I) sont présents à une concentration pouvant aller de 0,05 % à 20 % et de préférence de 0,5 % à 10 % en poids par rapport au poids total de la composition.

Ces compositions contiennent les ingrédients habituellement utilisés en cosmétique pour ce type de préparation. Ainsi elles peuvent contenir au moins un additif choisi parmi les alcools gras, les épaississants, les esters d'acides gras, les esters d'acides gras et de glycérol, les silicones (volatiles ou non, fonctionnalisés ou non), les tensioactifs, les parfums, les conservateurs, les filtres solaires, les protéines, les vitamines, les polymères, les huiles organiques ou inorganiques et tout autre additif classiquement utilisé dans le domaine cosmétique.

Les compositions de l'invention peuvent également se présenter sous toute forme de solutions ou de dispersions des dérivés tels que définis ci-dessus, éventuellement vésiculaires.

Toutes ces compositions sont préparées selon les méthodes usuelles connues de l'homme de l'art.

Un quatrième objet de l'invention concerne l'utilisation d'une composition telle que définie ci-dessus pour le traitement et/ou le soin du cheveu ou de la peau.

Un cinquième objet de l'invention porte sur un procédé de traitement cosmétique dans lequel on utilise une composition telle que définie ci-dessus, destiné notamment à améliorer le lissage et/ou le démêlage des cheveux.

Un sixième objet de l'invention porte sur un procédé de traitement cosmétique ou dermatologique dans lequel on utilise une composition telle que définie ci-dessus, destiné au traitement de la peau.

On va maintenant donner à titre d'illustration des exemples de préparation de dérivés de la sérine répondant à la formule (I), ainsi que des exemples de compositions les contenant. Ces exemples ne sauraient limiter en aucune façon la portée de l'invention.

### Exemple 1

### Préparation du 3-hydroxy-2-(N-hexadécyloxycarbonyl)-aminopropionate de dodécyle :

On met en oeuvre pour cette préparation le mode expérimental référencé B dans le texte.

Dans un cristallisoir de 30 ml, on introduit :
4,86 mmoles, (2 g), de 3-hydroxy-2-(N-hexadecyloxycarbonyl)-aminopropionate de potassium,
9,72 mmoles (2,32 ml) de bromododécane
0,04 g de chlorure de trioctylméthylammonium.

On irradie le mélange, pendant 3 minutes dans un four à micro-ondes (Menu Masters 3100i, 2450 MHz, puissance nominale 1400 watts).

On obtient après refroidissement une cire beige que l'on solubilise à environ 40°C dans 8 ml de méthanol par gramme de cire. Il subsiste un insoluble blanc (bromure de potassium), qui est éliminé par essorage.

Les filtrats sont refroidis à 4°C. Un précipité blanc apparaît qui est recueilli par essorage puis séché.

Le poids obtenu est de 1,8 g pour 2,63 g attendus, soit un rendement de 68%. On obtient des cristaux de couleur blanche dont les caractéristiques sont :

### Point de fusion (obtenu par Mettler FP 89) : F= 65°C (solvant = méthanol)

| Analyse élémentaire : C₃₂H₆₃ NO₅, 1,5 H₂O, PM = 569 | | | |
|---|---|---|---|
| | C | H | N |
| Calculé | 67,56 | 11,69 | 2,46 |
| Théorique (+1,5 H₂O) | 67,88 | 10,97 | 2,37 |

RMN ¹³C : conforme à la structure attendue.

### Exemple 2

### Préparation du 3-hydroxy-2-(N-hexadécyloxycarbonyl)-aminopropionate d'octyle.

On opère comme dans l'exemple 1, selon le procédé B, en mélangeant :
4,86 mmoles, (2g), de 3-hydroxy-2-(N-hexadecyloxycarbonyl)-aminopropionate de potassium,
9,72 mmoles (1,68 ml), de bromo-octane,
0,04 g de chlorure de trioctylméthylammonium.

On obtient un liquide ambré qui fige en refroidissant, que l'on solubilise à environ 40°C dans 7 ml de méthanol par gramme de produit. Il subsiste un insoluble blanc (bromure de potassium), qui est éliminé par essorage.

Les filtrats sont refroidis à 4°C. Un précipité blanc apparaît qui est recueilli par essorage puis séché.

Le poids obtenu est de 1,2 g pour 2,36g attendus, soit un rendement de 50%.

Les caractéristiques du produit sont :

### Point de fusion (obtenu par Mettler FP 89) : F= 55°C (solvant = méthanol)

| Analyse élémentaire : C₂₈H₅₅ NO₅, 1,5 H₂O, PM = 513 | | | |
|---|---|---|---|
| | C | H | N |
| Calculé | 65,59 | 11,4 | 2,73 |
| Théorique (+1,5 H₂O) | 65,50 | 10,77 | 2,92 |

RMN ¹³C : conforme à la structure attendue.

### Exemple 3

### Préparation du 3-hydroxy-2-(N-hexadécyloxycarbonyl)-aminopropionate d'hexadécyle

Ce produit est obtenu selon le procédé (A).

Dans un ballon de 500 ml, muni d'une agitation, d'un thermomètre et d'un réfrigérant ascendant, on introduit:
27 mmoles, (10g), d'acide 3-hydroxy-2-(N-hexadécyloxycarbonyl)-aminopropanoïque,
27 mmoles, (3g), de terbutanolate de potassium
100 ml de diméthylformamide.

On maintient à 20°C sous agitation pendant 1 heure.

On ajoute alors:
200 ml de N-méthylpyrrolidone
27 mmoles de bromure d'hexadécyle (8,2g).

On chauffe à 80°c pendant 2 heures et demi.

Les solvants sont éliminés sous pression réduite.

Le résidu obtenu est repris dans 120 ml d'acétate d'éthyle et 120 ml d'eau.

On obtient deux phases et un précipité que l'on recueille par essorage et que l'on reprend dans un mélange acétate d'éthyle/heptane additionné d'eau ammoniaquée.

On obtient deux phases et un précipité qui est essoré puis séché. Le poids de ce précipité est de 6,8 g.

Ce produit est ensuite purifié sur colonne de gel de silice.

Le poids de produit pur obtenu est de 4,1 g, correspondant à un rendement de 22,8%.

Ce produit a pour caractéristiques:

### Point de fusion (obtenu par Mettler FP 89) : F= 70°C (solvant = acétone)

| Analyse élémentaire : C₃₆H₇₁ NO₅, PM = 598 | | | |
|---|---|---|---|
| | C | H | N |
| Calculé | 72,31 | 11,97 | 2,34 |
| Théorique | 72,87 | 11,96 | 2,36 |

RMN ¹³C : conforme à la structure attendue.

### Exemple 4

### Préparation du 3-hydroxy-2-(N-hexadécyloxycarbonyl)-aminopropionate d'hexadécyle

On opère comme dans l'exemple 1, selon le procédé B, en mélangeant :
230 mmoles, (95g), de 3-hydroxy-2-(N-hexadécyloxycarbonyl)-aminopropionate de potassium.
230 mmoles, (69,9 g), de bromo- hexadécane,
1,9 g de chlorure de trioctylméthylammonium.

L'irradiation dure 4 minutes.

On obtient un liquide ambré qui fige en refroidissant.

Le solide obtenu est finement divisé, réempaté dans de l'eau, essoré, puis repris dans de l'acétone, essoré et séché.

On obtient ainsi 85 g de produit, des 137,5 attendus, soit un rendement de 61,8%, dont les caractéristiques sont:

### Point de fusion (obtenu par Mettler FP 89) : F= 70°C (solvant = acétone)

| Analyse élémentaire : C₃₆H₇₁ NO₅, PM = 598 | | | |
|---|---|---|---|
| | C | H | N |
| Calculé | 72,31 | 11,97 | 2,34 |
| Théorique | 72,98 | 12,05 | 2,36 |

RMN ¹³C : conforme à la structure attendue.

### Exemple 5

Il a été réalisé un test comparatif des effets sur le lissage du cheveu d'une composition cosmétique selon l'invention, contenant du 3-hydroxy-2-(N-hexadécyloxycarbonyl)-aminopropionate d'hexadécyle à 1 % dans l'isopropanol, par rapport à un témoin constitué d'isopropanol.

Ce test détermine le coefficient de frottement des cheveux par mesure de la force à appliquer à une masse témoin pour la faire glisser à vitesse constante sur deux cheveux tendus parallélement. La mesure est effectuée en faisant glisser la masse de la racine vers la pointe des cheveux (R → P) et inversement (P→R).

| **Cheveux** | **Traitement** | **Coefficient de frottement** | |
|---|---|---|---|
| | | **R→P** | **P→R** |
| Naturels | Témoin | 0,101 ± 0,003 | 0,127 ± 0,004 |
| | Composition selon l'invention | 0,084 ± 0,003 | 0,116 ± 0,006 |

L'application de la composition selon l'invention permet une nette diminution du coefficient de frottement, en particulier dans le sens racine → pointe (R→P), démontrant ainsi une amélioration du lissage ou du démêlage des cheveux.

### Exemple 6

On prépare l'après-shampooing ayant la composition suivante :

| | |
|---|---|
| 3-hydroxy-2-(N-hexadécyloxycarbonyl)-aminopropionate d'hexadécyle | 0,2 g |
| Chlorure de béhényl triméthylammonium (à 80 % dans un mélange eau/isopropanol 15/85 vendu sous le nom de DC 80 par la Société Toho) | 2 g |
| Eau qsp | 100 g |
| La solution est ajustée à un pH de 7,4. Après mélange des différents ingrédients, on obtient une solution fluide laiteuse. Cette solution est appliquée sur la chevelure après un shampooing pendant quelques minutes, puis est rincée à l'eau. On obtient ainsi une chevelure plus facilement coiffable. | |

### Exemple 7

On prépare la crème traitante ayant la composition suivante :

| | |
|---|---|
| Ceteareth-30 (selon la nomenclature CTFA) | 2,5 g * |
| Hydroxyéthylcellulose | 0,5 g |
| Chlorure de béhényl triméthylammonium (à 80 % dans un mélange eau/isopropanol 15/85 vendu sous le nom de DL 80 par la Société Toho) | 1,5 g |
| 3-hydroxy-2-(N-hexadécyloxycarbonyl)-aminopropionate d'hexadécyle | 0,5 g |
| Eau qsp | 100 g |
| La solution est ajustée à pH 6. Après mélange on obtient une crème de couleur blanche qui s'applique facilement sur les cheveux et leur confère un démêlage facile. | |

| | |
|---|---|
| * ^{:} exprimé en poids de matière active dans les exemples 7 et 8. | |

### Exemple 8 :

On prépare le shampooing-crème suivant :

| | |
|---|---|
| Laurylether sulfate de sodium, oxyéthyléné à 2,2 moles | 8 g * |
| Cocobétaïne (selon la nomenclature CTFA) | 4 g * |
| Distéarate de glycol | 1 g |
| Cocoyl Diethanolamide | 1 g |
| 3-hydroxy-2-(N-hexadécyloxycarbonyl)-aminopropionate d'hexadécyle | 0,2 g |
| Conservateur, colorant, parfum et eau : qsp | 100 g |
| Le pH de la solution est ajusté 7,5. Après mélange on obtient un shampooing-crème nacré, confortable à l'application ayant une mousse douce et apportant aux cheveux une facilité de démêlage et un effet de lissage. | |

| | |
|---|---|
| * ^{:} exprimé en poids de matière active dans les exemples 7 et 8. | |

## Revendications

1. Dérivés de la sérine, caractérisés par le fait qu'ils répondent à la formule générale : 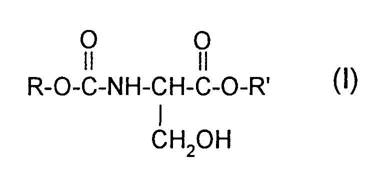 dans laquelle R et R', identiques ou différents représentent un radical hydrocarboné linéaire ou ramifié, ayant de 8 à 30 atomes de carbone, et leurs isomères optiques D ou L ou leurs mélanges.

2. Dérivés de la sérine selon la revendication 1, caractérisés par le fait que R et R', identiques ou différents sont des radicaux saturés ou qui comportent une double liaison.

3. Dérivés de la sérine selon l'une quelconque des revendications 1 et 2, caractérisés par le fait que R et R', identiques ou différents, contiennent de préférence entre 8 et 18 atomes de carbone.

4. Dérivés de la sérine selon l'une quelconque des revendications précédentes, caractérisés par le fait qu'ils sont choisis dans le groupe constitué du :
- 3-hydroxy-2-(N-hexadécyloxycarbonyl)-aminopropionate de dodécyle,
- 3-hydroxy-2-(N-hexadécyloxycarbonyl)-aminopropionate d'octyle,
- 3-hydroxy-2-(N-hexadécyloxycarbonyl)-aminopropionate d'hexadécyle,
- 3-hydroxy-2-(N-2-éthyl-hexyloxycarbonyl)-aminopropionate de dodécyle,
- 3-hydroxy-2-(N-2-éthyl-hexyloxycarbonyl)-aminopropionate d'octyle,
- 3-hydroxy-2-(N-2-éthyl-hexyloxycarbonyl)-aminopropionate d'hexadécyle,
- 3-hydroxy-2-(N-dodécyloxycarbonyl)-aminopropionate de dodécyle,
- 3-hydroxy-2-(N-dodécyloxycarbonyl)-aminopropionate d'octyle,
- 3-hydroxy-2-(N-dodécyloxycarbonyl)-aminopropionate d'hexadécyle,
- 3-hydroxy-2-(N-oleyloxycarbonyl)-aminopropionate de dodécyle,
- 3-hydroxy-2-(N-oleyloxycarbonyl)-aminopropionate d'octyle,
- 3-hydroxy-2-(N-oleyloxycarbonyl)-aminopropionate d'hexadécyle.

5. Dérivés de la sérine selon l'une quelconque des revendications précédentes, caractérisés par le fait qu'ils sont choisis préférentiellement dans le groupe constitué du :
- 3-hydroxy-2-(N-hexadécyloxycarbonyl)-aminopropionate de dodécyle,
- 3-hydroxy-2-(N-hexadécyloxycarbonyl)-aminopropionate d'octyle,
- 3-hydroxy-2-(N-hexadécyloxycarbonyl)-aminopropionate d'hexadécyle.

6. Procédé de préparation des dérivés selon l'une quelconque des revendications précédentes, caractérisé par le fait qu'il consiste à faire réagir, en présence d'un agent de transfert de phase, un sel de N-liposérine répondant à la formule : 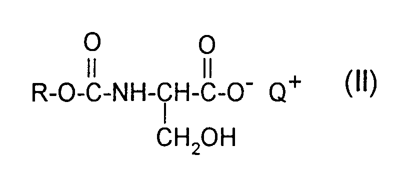 et un dérivé hydrocarboné activé répondant à la formule :
R'X (III)
dans laquelle R et R', identiques ou différents représentent un radical hydrocarboné linéaire ou ramifié, ayant de 8 à 30 atomes de carbone ;
Q⁺ est un cation organique ou minéral ;
X un groupement activant.

7. Procédé selon la revendication 6, caractérisé par le fait que R et R', identiques ou différents, sont des radicaux saturés ou qui comportent une double liaison.

8. Procédé selon l'une quelconque des revendications 6 ou 7, caractérisé par le fait que X est un atome d'halogène ou un radical mésyloxy, brosyloxy ou tosyloxy.

9. Procédé selon l'une quelconque des revendications 6 à 8, caractérisé par le fait que l'agent de transfert de phase est un agent de transfert de phase solide/liquide.

10. Procédé selon l'une quelconque des revendications 6 à 9, caractérisé par le fait que l'agent de transfert de phase est un dérivé d'ammonium quaternaire.

11. Composition cosmétique ou dermatologique, caractérisée par le fait qu'elle contient au moins un dérivé selon l'une quelconque des revendications 1 à 5.

12. Composition cosmétique ou dermatologique selon la revendication 11, caractérisée par le fait qu'elle contient entre 0,05 % et 20 % de dérivés de la sérine en poids par rapport au poids total de la composition.

13. Composition cosmétique ou dermatologique selon l'une quelconque des revendications 11 ou 12, caractérisée par le fait qu'elle contient de préférence entre 0,5% et 10% de dérivés de la sérine en poids par rapport au poids total de la composition.

14. Utilisation d'une composition cosmétique selon l'une quelconque des revendications 11 à 13 pour le traitement ou le soin des cheveux.

15. Utilisation d'une composition cosmétique selon l'une quelconque des revendications 11 à 13 pour le traitement ou le soin de la peau.

16. Procédé de traitement cosmétique du cheveu, caractérisé par le fait que l'on utilise une composition cosmétique selon l'une quelconque des revendications 11 à 13.

17. Procédé de traitement cosmétique de la peau, caractérisé par le fait que l'on utilise une composition cosmétique ou dermatologique selon l'une quelconque des revendications 11 à 13.

## Claims

1. Serine derivatives, characterized in that they correspond to the general formula: 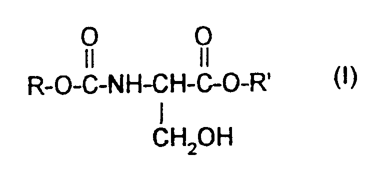 in which R and R', which are identical or different, represent a linear or branched hydrocarbon radical having from 8 to 30 carbon atom
and their D or L optical isomers or their mixtures.

2. Serine derivatives according to Claim 1, characterized in that R and R', which are identical or different, are saturated radicals or radicals which contain a double bond.

3. Serine derivatives according to either of Claims 1 and 2, characterized in that R and R', which are identical or different, preferably contain between 8 and 18 carbon atoms.

4. Serine derivatives according to any one of the preceding claims, characterized in that they are chosen from the group consisting of:
- dodecyl 3-hydroxy-2-[N-(hexadecyloxycarbonyl)amino]propionate,
- octyl 3-hydroxy-2-[N-(hexadecyloxycarbonyl)amino]propionate,
- hexadecyl 3-hydroxy-2-[N-(hexadecyloxycarbonyl)amino]propionate,
- dodecyl 3-hydroxy-2-[N-(2-ethylhexyloxycarbonyl)amino]propionate,
- octyl 3-hydroxy-2-[N-(2-ethylhexyloxycarbonyl)amino]propionate,
- hexadecyl 3-hydroxy-2-[N-(2-ethylhexyloxycarbonyl)amino]propionate,
- dodecyl 3-hydroxy-2-[N-(dodecyloxycarbonyl)amino]propionate,
- octyl 3-hydroxy-2-[N-(dodecyloxycarbonyl)amino]propionate,
- hexadecyl 3-hydroxy-2-[N-(dodecyloxycarbonyl)amino]propionate,
- dodecyl 3-hydroxy-2-[N-(oleyloxycarbonyl)amino]propionate,
- octyl 3-hydroxy-2-[N-(oleyloxycarbonyl)amino]propionate,
- hexadecyl 3-hydroxy-2-[N-(oleyloxycarbonyl)amino]propionate.

5. Serine derivatives according to any one of the preceding claims, characterized in that they are preferentially chosen from the group consisting of:
- dodecyl 3-hydroxy-2-[N-(hexadecyloxycarbonyl)amino]propionate,
- octyl 3-hydroxy-2-[N-(hexadecyloxycarbonyl)amino]propionate,
- hexadecyl 3-hydroxy-2-[N-(hexadecyloxycarbonyl)amino]propionate.

6. Process for the preparation of the derivatives according to any one of the preceding claims, characterized in that it consists in reacting, in the presence of a phase transfer agent, an N-liposerine salt corresponding to the formula: 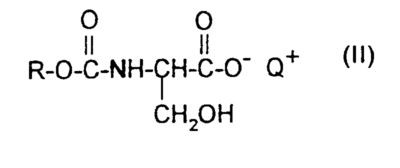 and an activated hydrocarbon derivative corresponding to the formula:
R'X (III)
in which R and R', which are identical or different, represent a linear or branched hydrocarbon radical having from 8 to 30 carbon atoms;
Q⁺ is an organic or inorganic cation;
X an activating group.

7. Process according to Claim 6, characterized in that R and R', which are identical or different, are saturated radicals or radicals which contain a double bond.

8. Process according to either of Claims 6 and 7, characterized in that X is a halogen atom or a mesyloxy, brosyloxy or tosyloxy radical.

9. Process according to any one of Claims 6 to 8, characterized in that the phase transfer agent is a solid/liquid phase transfer agent.

10. Process according to any one of Claims 6 to 9, characterized in that the phase transfer agent is a quaternary ammonium derivative.

11. Cosmetic or dermatological composition, characterized in that it contains at least one derivative according to any one of Claims 1 to 5.

12. Cosmetic or dermatological composition according to Claim 11, characterized in that it contains between 0.05 % and 20 % of serine derivatives by weight with respect to the total weight of the composition.

13. Cosmetic or dermatological composition according to either of Claims 11 and 12, characterized in that it preferably contains between 0.5 % and 10 % of serine derivatives by weight with respect to the total weight of the composition.

14. Use of a cosmetic composition according to any one of Claims 11 to 13 for treating or caring for the hair.

15. Use of a cosmetic composition according to any one of Claims 11 to 13 for treating or caring for the skin.

16. Process for the cosmetic treatment of the hair, characterized in that a cosmetic composition according to any one of Claims 11 to 13 is used.

17. Process for the cosmetic treatment of the skin, characterized in that a cosmetic or dermatological composition according to any one of Claims 11 to 13 is used.

## Patentansprüche

1. Serinderivate, dadurch gekennzeichnet, daß sie der allgemeinen Formel (I) 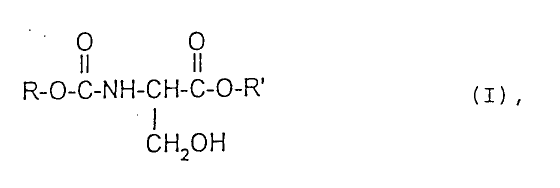 entsprechen,
worin R und R', die gleich oder voneinander verschieden sind, eine geradkettige oder verzweigte Kohlenwasserstoffgruppe mit 8 bis 30 Kohlenwasserstoffatomen bedeuten,
einschließlich ihrer optischen D- und L-Isomeren sowie deren Gemischen.

2. Serinderivate nach Anspruch 1, dadurch gekennzeichnet, daß R und R', die gleich oder voneinander verschieden sind, gesättigte Gruppen oder Gruppen bedeuten, die eine Doppelbindung enthalten.

3. Serinderivate nach Anspruch 1 oder 2,
dadurch gekennzeichnet, daß R und R', die gleich oder voneinander verschieden sind, vorzugsweise 8 bis 18 Kohlenstoffatome enthalten.

4. Serinderivate nach einem der vorhergehenden Ansprüche, dadurch gekennzeichnet, daß sie ausgewählt sind unter:
- 3-Hydroxy-2-(N-hexadecyloxycarbonyl)-aminopropionsäuredodecylester,
- 3-Hydroxy-2-(N-hexadecyloxycarbonyl)-aminopropionsäureoctylester,
- 3-Hydroxy-2-(N-hexadecyloxycarbonyl)-aminopropionsäurehexadecylester,
- 3-Hydroxy-2-(N-2-ethylhexyloxycarbonyl)-aminopropionsäuredodecylester,
- 3-Hydroxy-2-(N-2-ethylhexyloxycarbonyl)-aminopropionsäureoctylester,
- 3-Hydroxy-2-(N-2-ethylhexyloxycarbonyl)-aminopropionsäurehexadecylester,
- 3-Hydroxy-2-(N-dodecyloxycarbonyl)-aminopropionsäuredodecylester,
- 3-Hydroxy-2-(N-dodecyloxycarbonyl)-aminopropionsäureoctylester,
- 3-Hydroxy-2-(N-dodecyloxycarbonyl)-aminopropionsäurehexadecylester,
- 3-Hydroxy-2-(N-oleyloxycarbonyl)-aminopropionsäuredodecylester,
- 3-Hydroxy-2-(N-oleyloxycarbonyl)-aminopropionsäureoctylester und
- 3-Hydroxy-2-(N-oleyloxycarbonyl)-aminopropionsäurehexadecylester.

5. Serinderivate nach einem der vorhergehenden Ansprüche, dadurch gekennzeichnet, daß sie vorzugsweise ausgewählt sind unter:
- 3-Hydroxy-2-(N-hexadecyloxycarbonyl)-aminopropionsäuredodecylester,
- 3-Hydroxy-2-(N-hexadecyloxycarbonyl)-aminopropionsäureoctylester und
- 3-Hydroxy-2-(N-hexadecyloxycarbonyl)-aminopropionsäurehexadecylester,

6. Verfahren zur Herstellung der Derivate nach einem der vorhergehenden Ansprüche, dadurch gekennzeichnet, daß es darin besteht, in Gegenwart eines Phasentransfermittels ein N-Liposerinsalz der folgenden Formel (II) 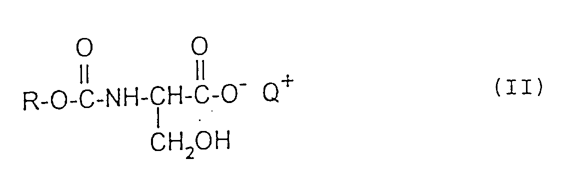 mit einem aktivierten Kohlenwasserstoffderivat der Formel (III)
R'X (III)
umzusetzen, worin bedeuten:
R und R', die gleich oder voneinander verschieden sind, eine geradkettige oder verzweigte Kohlenwasserstoffgruppe mit 8 bis 30 Kohlenstoffatomen,
Q⁺ ein organisches oder anorganisches Kation und
X eine aktivierende Gruppe.

7. Verfahren nach Anspruch 6, dadurch gekennzeichnet, daß R und R', die gleich oder voneinander verschieden sind, gesättigte Gruppen oder Gruppen bedeuten, die eine Doppelbindung enthalten.

8. Verfahren nach Anspruch 6 oder 7, dadurch gekennzeichnet, daß X ein Halogenatom oder eine Mesyloxy-, Brosyloxy- oder Tosyloxy-Gruppe bedeutet.

9. Verfahren nach einem der Ansprüche 6 bis 8, dadurch gekennzeichnet, daß das Phasentransfermittel ein Fest/Flüssig-Phasentransfermittel ist.

10. Verfahren nach einem der Ansprüche 6 bis 9, dadurch gekennzeichnet, daß das Phasentransfermittel ein quaternäres Ammoniumderivat ist.

11. Kosmetische oder dermatologische Zusammensetzung, dadurch gekennzeichnet, daß sie mindestens ein Derivat nach einem der Ansprüche 1 bis 5 enthält.

12. Kosmetische oder dermatologische Zusammensetzung nach Anspruch 11, dadurch gekennzeichnet, daß sie 0,05 bis 20 Gew.-% Serinderivate, bezogen auf das Gesamtgewicht der Zusammensetzung, enthält.

13. Kosmetische oder dermatologische Zusammensetzung nach einem der Ansprüche 11 oder 12, dadurch gekennzeichnet, daß sie vorzugsweise 0,5 bis 10 Gew.-% Serinderivate, bezogen auf das Gesamtgewicht der Zusammensetzung, enthält.

14. Verwendung einer kosmetischen Zusammensetzung nach einem der Ansprüche 11 bis 13 zur Behandlung oder zur Pflege der Haare.

15. Verwendung einer kosmetischen Zusammensetzung nach einem der Ansprüche 11 bis 13 zur Behandlung oder zur Pflege der Haut.

16. Verfahren zur kosmetischen Behandlung der Haare, dadurch gekennzeichnet, daß eine kosmetische Zusammensetzung nach einem der Ansprüche 11 bis 13 verwendet wird.

17. Verfahren zur kosmetischen Behandlung der Haut, dadurch gekennzeichnet, daß eine kosmetische oder dermatologische Zusammensetzung nach einem der Ansprüche 11 bis 13 verwendet wird.
